# EUROPÄISCHE PATENTSCHRIFT

(11) **EP 3 484 566 B1**
(45) Veröffentlichungstag und Bekanntmachung des Hinweises auf die Patenterteilung: **12.02.2020**
(21) Anmeldenummer: 17740293.0
(22) Anmeldetag: 13.07.2017
(51) Int. Cl.: A61M 16/18, A61M 16/00

(54) **DOSIERVORRICHTUNG ZUM EINBRINGEN EINES ANÄSTHESIEMITTELS IN EINEN ATEMGASSTROM**
METERING APPARATUS FOR INTRODUCING AN ANAESTHETIC AGENT INTO A RESPIRATORY GAS FLOW
DISPOSITIF DE DOSAGE PERMETTANT D'INTRODUIRE UN AGENT ANESTHÉSIQUE DANS UN FLUX DE GAZ RESPIRATOIRE

(30) Priorität: 14.07.2016 DE 102016008493
(43) Veröffentlichungstag der Anmeldung: 22.05.2019
(73) Patentinhaber: Drägerwerk AG & Co. KGaA, 23558 Lübeck (DE)
(72) Erfinder: WRUCK, Norbert, 23568 Lübeck (DE); RIECKE, Michael, 23564 Lübeck (DE)
(86) Internationale Anmeldenummer: PCT/EP2017/000834
(87) Internationale Veröffentlichungsnummer: WO 2018/010845

(56) Entgegenhaltungen:
- EP-A1- 3 034 123
- DE-A1- 3 116 951
- DE-A1- 3 234 474
- DE-A1- 4 116 512
- DE-A1- 19 823 959
- DE-A1- 19 914 977

## Beschreibung

Die Erfindung betrifft eine Dosiervorrichtung zum Einbringen eines Anästhesiemittels in einen Atemgasstrom, die einen Strömungskanal für den das Anästhesiemittel aufweisenden Atemgasstrom, eine Steuerungseinheit und einen ersten Temperatursensor aufweist, wobei die Dosiervorrichtung eine Zuführeinrichtung mit einer im Strömungskanal angeordneten Verdunstungsoberfläche für Anästhesiemittel aufweist, wobei der erste Temperatursensor die Temperatur der Verdunstungsoberfläche erfasst und ein erstes Temperatursignal an die Steuerungseinheit übermittelt.

Die Narkotisierung von Patienten in Operationssälen erfolgt mittels Anästhesiemitteln. Die Patienten werden dabei von Maschinen mit Atemluft versorgt. Diese Maschinen erzeugen ein sogenanntes Atemgas, dem das Anästhesiemittel beigemischt wird. Die Konzentration des Anästhesiemittels muss dabei so gewählt werden, dass der Patient narkotisiert wird, ohne dass bleibende Schäden oder gar der Tod eintreten.

Es existieren trägergasabhängige und trägergasunabhängige Systeme. Bei trägergasunabhängigen Systemen wird das Anästhesiemittel flüssig oder dampfförmig abgemessen und dann dem Atemgasstrom zugeführt.

Bei trägergasabhängigen Systemen wird das Anästhesiemittel in den Atemgasstrom z. B. durch Verdunsten eingetragen. Die in das Trägergas beigemischte Dosis des Anästhesiemittels wird dabei durch den Trägergasstrom und die Temperatur bzw. den Dampfdruck des Anästhesiemittels in einer Verdunsterkammer beeinflusst.

Es sind Anästhesiemitteldosierer bekannt, die pneumatisch-mechanisch an einer Anästhesiemaschine angebunden sind. Dabei werden nur mechanische Funktionsteile zur Dosierung verwendet, wobei feinmechanische Laminarflowelemente (z. B. Bypass- und Dosierspalte) benutzt werden, deren Einstellung mittels mechanischer Temperaturkompensationselemente, die auf thermischer Längenänderung basieren, vorgenommen wird.

Bei diesen Vorrichtungen ist eine aufwändige manuelle Kalibration der Bypass- und Dosierspalte für eine präzise Einhaltung der geforderten Abgabekonzentration notwendig. Weiter werden große thermische Speichermassen benötigt, um Temperaturschwankungen möglichst gering zu halten und um den Sättigungszustand für das Atemgas zu erreichen bzw. zu erhalten. Der Atemgasstrom wird dabei so gewählt, dass er bei Durchströmung durch die Vorrichtung mit Anästhesiemittel vollständig gesättigt wird.

Die mechanischen und feinmechanischen Elemente sind nicht elektrisch ansteuerbar, sodass es nicht möglich ist, die Abgabekonzentration des Anästhesiemittels an den Atemgasstrom bei unterschiedlichen Temperaturen automatisch zu regeln. Weiter können die Gasflüsse und die Betriebsdauern der mechanischen Elemente nicht gemessen werden. Es ist weiter bekannt, die Anästhesiemittelkonzentration im Atemgas mit optischen Mitteln zu erfassen. Allerdings ist diese optische Messung der Anästhesiemittelkonzentration sehr teuer, fehleranfällig und wartungsintensiv.

DE 41 16 512 A1 offenbart einen Narkosemittelverdunster mit einer Verdunsterkammer, welche von einem Verdunsterkammer-Strom von einem Verdunsterkammer-Einlass zu einem Verdunsterkammer-Auslass durchströmt wird und mit in der Verdunsterkammer kaskadenförmig angeordneten, Narkosemittel speichernden Verdunsterelementen zur Anreicherung des Verdunsterkammer-Stroms mit Narkosemitteldampf und einer Thermostateinrichtung mit einem Temperatursensor am Verdunsterkammer-Auslass.

EP 3 034 123 A1 offenbart eine Narkosemittelverdunstereinheit mit einem Narkosemittelbehälter zur Aufnahme von flüssigem Narkosemittel, einer Gasleitung mit einer offenporigen, porösen Leitungswandung zum Leiten von Gas und zum Anreichern des Gases mit Narkosemittel, und einem narkosemittelleitenden Docht, der sich von einem Innenraum des Narkosemittelbehälters zu der Leitungswandung erstreckt, um die Leitungswandung mit Narkosemittel aus dem Narkosemittelbehälter zu versorgen. Die Narkosemittelverdunstungseinheit weist eine Heizung zum Beheizen der Gasleitung auf. Ein Temperatursensor zu der Erfassung von einer Temperatur des Gases in der Gasleitung ist vorgesehen.

DE 198 23 959 A1 und DE 199 14 977 A1 beziehen sich auf Techniken zum Bestimmen der Konzentration eines Dampfes (z.B. eines Anästhetikums) in einem Gasstrom und insbesondere auf Vorrichtungen und Verfahren zum Bestimmen der Feuchtigkeit in einem Luftstrom als Ergebnis einer Differenz zwischen einer Feuchttemperatursensor und einer Trockentemperatursensor.

DE 31 16 951 A1 und DE 32 34 474 A1 offenbaren eine Vorrichtung zur Beimischung flüssiger Narkosemittel in das dem Patienten zuzuführende Atemgas mit einer Steuer- und Auswerteschaltung und Einrichtungen zur geregelten Zuführung des Atemgases sowie des Narkosemittels, mit einer Mischkammer in einem wärmeisolierten Gehäuse mit einem Einlaß für das Atemgas vor, sowie mit einem Auslaß für das Atemgas plus dem verdunsteten Narkosemittel hinter der Mischkammer, wobei der Einlaß einen Einlaßtemperaturfühler und der Auslaß einen Auslaßtemperaturfühler besitzen.

Aus der GB 2 425 728 ist es bekannt, die Temperatur des Anästhesiemittels mit einem außen an einem Anästhesiemitteltank angeordneten Messelement zu überwachen. Die von dem Messelement gemessene Temperatur gibt einen ungefähren Anhaltspunkt für den Mittelwert des Dampfdruckes des Anästhesiemittels im Tank wieder. Für die Berechnung der Konzentration des Anästhesiemittels müssen aus tankspezifischen Messungen Annahmen getroffen werden, die in das Ergebnis der Konzentrationsbestimmung einfließen müssen. Diese Methode ist ungenau, da sie auf ungenaue Annahmen gestützt ist.

Aufgabe der Erfindung ist es daher, eine Vorrichtung zu schaffen, die eine genaue, kostengünstige, elektronische Ansteuerung für einen Anästhesiemitteldosierer bereitstellt.

Die Aufgabe wird gelöst durch die Merkmale der unabhängigen Ansprüche. Vorteilhafte Weiterbildungen sind Gegenstand der abhängigen Ansprüche.

Bei einer eingangs genannten Dosiervorrichtung für Anästhesiemittel in einem Atemgasstrom ist dabei erfindungsgemäß vorgesehen, dass die Dosiervorrichtung einen zweiten Temperatursensor aufweist, der die Temperatur des Atemgasstroms im Strömungskanal erfasst und als zweites Temperatursignal an die Steuerungseinheit übermittelt, wobei die Steuerungseinheit zum Steuern einer Anästhesiemittelkonzentration mittels des ersten und zweiten Temperatursignals ausgebildet ist.

Mittels der Erfindung wird somit eine "feuchte" Temperatur bestimmt, die auf der Verdunstungsoberfläche für Anästhesiemittel der Zuführeinrichtung gemessen wird und eine "trockene" Temperatur bestimmt, die im Atemgasstrom des Strömungskanals gemessen wird. Da die Verdunstungsoberfläche der Zuführeinrichtung in dem Atemgasstrom angeordnet ist, wird sie ständig von Atemgas umströmt. Solange das Atemgas zu 100 % mit Anästhesiemittel gesättigt ist, verdunstet kein weiteres Anästhesiemittel von der Verdunstungsoberfläche. An der Verdunstungsoberfläche wird sich daher eine Temperatur einstellen, die der Temperatur des Atemgasstroms entspricht, der zu 100 % mit Anästhesiemittel gesättigt ist.

Sobald der Atemgasstrom auf Grund einer Zustandsänderung nicht mehr zu 100 % mit Anästhesiemittel gesättigt ist, nimmt der Atemgasstrom, der in Kontakt mit der Verdunstungsoberfläche steht, weiteres Anästhesiemittel über Verdunstung auf. Der Atemgasstrom kühlt dabei die Verdunstungsoberfläche auf eine Temperatur ab, die die adiabate Temperatur genannt wird. Zu dieser Temperatur gehört eine 100-prozentige Sättigung des Atemgasstroms mit Anästhesiemittel. Die Temperatur der feuchten Verdunstungsoberfläche entspricht daher der Temperatur des Atemgases mit vollständiger Anästhesiemittelsättigung bevor ein Nebelzustand erreicht wird.

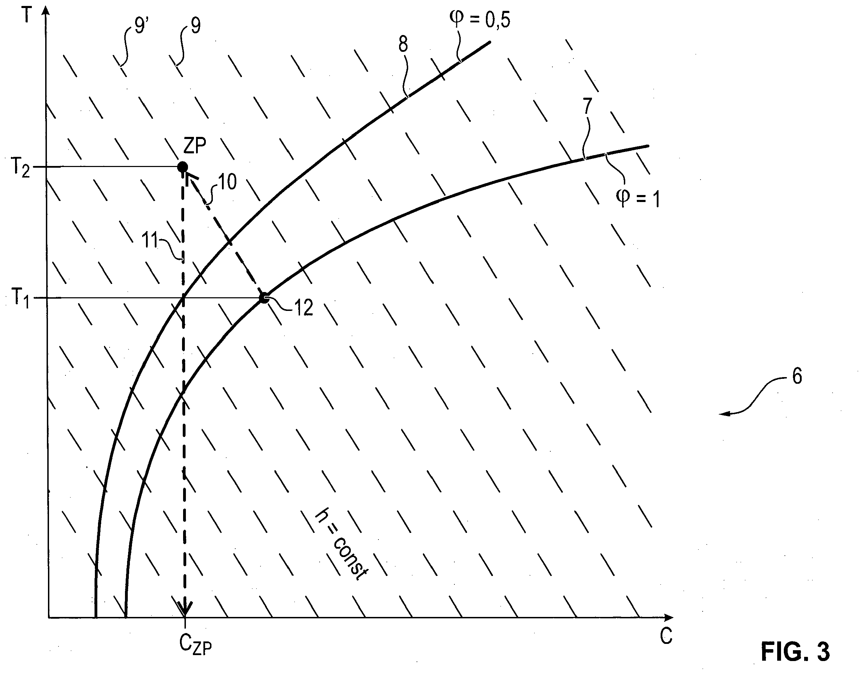

Mittels des ersten und zweiten Temperatursignals kann damit die Konzentration des Anästhesiemittels im Atemgasstrom bestimmt werden, beispielsweise analog dem Messprinzip eines Psychrometers oder beispielsweise mittels eines h+,x-Diagramms nach Mollier wie zu Figur 3 näher beschrieben. Auch bei schwankenden Temperaturen kann ohne eine Kalibration eine genaue Bestimmung der Konzentration erfolgen, da die Schwankungen der Temperatur durch die Erfassung des ersten und zweiten Temperatursignals bei der Bestimmung der Anästhesiemittelkonzentration berücksichtigt werden. Die Bestimmung der Anästhesiemittelkonzentration im Atemgasstrom mittels der Messung des ersten und zweiten Temperatursignals ist daher universell einsetzbar und kalibrationsfrei.

Weiter werden dadurch keine thermischen Speichermassen und keine teuren mechanischen Temperaturkompensationselemente mehr benötigt. Anästhesiemitteldosierer werden dadurch leichter und kostengünstiger. Weiter kann mit der Erfindung eine elektronische Regelung des Anästhesiemitteldosierers erfolgen, so dass mit der bekannten Anästhesiemittelkonzentration am Ausgang des Anästhesiemitteldosierers eine automatische Steuerung der Anästhesiemittelkonzentration im Atemgasstrom zum Patienten erfolgen kann. Die Steuerung der Konzentration kann dabei an einer beliebigen Stelle nach der Messung z. B. durch Verdünnung mit frischem Atemgas bewirkt werden. Alternativ kann zur Steuerung der Anästhesiemittelkonzentration im Atemgasstrom die Steuerungseinheit z. B. die Temperatur des Atemgasstroms oder des Anästhesiemittels oder die Geschwindigkeit des Atemgasstroms beeinflussen. Der erste und zweite Temperatursensor können in einem Strömungskanal stromab einer Verdunsterkammer angeordnet sein. Die Verdunstungsoberfläche ist in diesem Fall stromab einer ersten Verdunstungsoberfläche der Verdunsterkammer in einem nachgeordneten Strömungskanal angeordnet und dient primär der Bestimmung der feuchten Temperatur durch den ersten Temperatursensor, wobei sie durch die Zuführeinrichtung mit Anästhesiemittel befeuchtet wird. Der erste und zweite Temperatursensor können beispielsweise auch innerhalb der Verdunsterkammer angeordnet sein, vorzugsweise stromab der ersten Hälfte des Weges des Strömungskanals durch die Verdunsterkammer hindurch. Beispielsweise in einem Ausgangsbereich der Verdunsterkammer. In diesen Fällen ist die Verdunstungsoberfläche der Zuführeinrichtung in der Verdunsterkammer angeordnet und kann mit einer Verdunstungsoberfläche der Verdunstungskammer identisch sein.

Bevor auf vorteilhafte Weiterbildungen näher eingegangen wird, folgt eine Definition eines Begriffs.

Unter einem Strömungskanal seien zum einen ein gemeinsamer Strömungskanal und zum anderen auch ein geteilter Strömungskanal verstanden, wobei die beiden Teile des geteilten Strömungskanals miteinander fluidkommunizierend verbunden sind. Der erste und der zweite Temperatursensor können die Erfassung der Temperaturen daher auch in verschiedenen Teilbereichen eines Strömungskanalsystems durchführen.

Vorteilhafterweise weist die Steuerungseinheit ein Bestimmungsmodul auf, das aus dem erfassten ersten und zweiten Temperatursignal einen Zustandspunkt des Anästhesiemittels bestimmt, wobei der Zustandspunkt einer Anästhesiemittelkonzentration im Atemgasstrom zugeordnet ist. Die Bestimmung kann mittels eines Zustandsdiagramms erfolgen, das dabei im Bestimmungsmodul hinterlegt ist. Auf diese Weise kann die Steuerungseinheit mittels der Messung der beiden Temperaturen einfach und schnell die Konzentration des Anästhesiemittels im Atemgasstrom ermitteln.

Es kann vorteilhaft vorgesehen sein, dass die Steuerungseinheit ein Berechnungsmodul aufweist, das zum Bestimmen einer Differenz zwischen dem ersten und zweiten Temperatursignal ausgebildet ist, wobei die Steuerungseinheit zum Steuern der Anästhesiemittelkonzentration mittels des ersten und zweiten Temperatursignals ausgebildet ist.

In diesem Fall kann eine Temperaturdifferenz zwischen der ersten Temperatur und der zweiten Temperatur ermittelt werden. Wenn eine Temperaturdifferenz ermittelt wird, zeigt dies an, dass der Atemgasstrom nicht zu 100 % mit Anästhesiemittel gesättigt ist. Die Steuerung kann beispielsweise auf Basis dieser Temperaturdifferenz u. a. Maßnahmen ergreifen, die den Atemgasstrom zu einer 100 % Sättigung mit Anästhesiemittel zurückführen und beispielsweise gleichzeitig die zweite Temperatur auf einen gewünschten Wert regeln und/oder halten, um eine Anästhesiemittelkonzentration einzustellen. Alternativ oder zusätzlich kann die Steuerung derart ausgebildet sein, dass diese zur Bestimmung der Anästhesiemittelkonzentration im Atemgasstrom die Differenz in eine Psychrometerformel einfließen lässt.

Durch die Erfassung der "trockenen" und "feuchten" Temperatur des Anästhesiemittels im Strömungskanal, kann die Anästhesiemittelkonzentration mit einer hohen Genauigkeit bestimmt werden.

Mit Vorteil weist die Steuerungseinheit ein Berechnungsmodul auf, das zum Bestimmen einer Differenz zwischen den Temperatursignalen ausgebildet ist, wobei die Steuerungseinheit eine Regelungseinheit aufweist, die zum Regeln des Differenzbetrags auf ein Minimum ausgebildet ist.

Die Steuerungseinheit weist mit Vorteil einen Sollwerteingang für ein Sollwertsignal für die Anästhesiemittelkonzentration im Atemgasstrom und eine Regelungseinheit auf, die zum Regeln der Anästhesiemittelkonzentration in einer Patientenzuleitung auf das Sollwertsignal ausgebildet ist.

Über den Sollwerteingang kann ein Benutzer der Dosiervorrichtung einen Sollwert für die Anästhesiemittelkonzentration an die Steuerungseinheit übermitteln.

Die Dosiervorrichtung weist zweckmäßigerweise ein Ausström-Ventil auf, das fluidkommunizierend mit dem Strömungskanal verbunden ist und durch die Steuerungseinheit gesteuert wird.

Dabei kann die Steuerungseinheit ein Steuersignal an das Ausström-Ventil übermitteln. Das Ausström-Ventil kann weiter den Strömungskanal öffnen und verschließen. In Abhängigkeit der ermittelten Anästhesiemittelkonzentration im Atemgasstrom, kann die Größe der Ventilöffnung durch die Steuereinheit verstellt werden.

Bei dem Ausström-Ventil kann es sich zweckmäßigerweise um ein Proportional-Ventil handeln, das vorzugsweise als Konusventil ausgebildet sein kann.

Damit ist eine graduelle Änderung des Atemgas-Durchflusses im Strömungskanal ermöglicht. Der Atemgasstrom kann mit dem Proportional-Ventil mit hoher Genauigkeit eingestellt werden.

Es ist weiter vorteilhaft, wenn die Temperatursensoren als elektrische Sensoren, gekapselte oder ungekapselte Thermoelemente, als Widerstandselemente oder als NTC-Widerstände (Negative Temperature Coefficient) ausgebildet sind.

Weiter können die Temperatursensoren mit Vorteil als kontaktlos messende Infrarotdetektoren ausgebildet sein. Alternativ können sie einstückig ausgebildet sein, wobei sie als ein kontaktlos messender ortsauflösender Infrarotsensor ausgebildet sind.

Es ist weiter zweckmäßig, dass die Dosiervorrichtung eine Verdunsterkammer aufweist, in der stromaufwärts der Verdunstungsoberfläche ein Anästhesiemittel-Reservoir angeordnet ist, wobei die Zuführeinrichtung mit dem Anästhesiemittel-Reservoir fluidkommunizierend verbunden ist.

Dabei wird der Atemgasstrom durch die Verdunsterkammer geleitet, bevor er in den Strömungskanal gelangt. Das Anästhesiemittel-Reservoir kann als ein zu einer Seite offener Tank ausgestaltet sein. In dem Anästhesiemittel-Reservoir ist ein Anästhesiemittel gelagert. Das Anästhesiemittel ist in der Regel eine flüchtige Substanz und in flüssiger Form vorhanden. Die Oberfläche des Anästhesiemittels an der offenen Seite des Tanks wird von dem Atemgasstrom überstrichen und fungiert als Haupt-Verdunstungsoberfläche, die primär dem Einbringen des Anästhesiemittels in den Atemgasstrom dient. Dabei verdunstet das Anästhesiemittel in den Atemgasstrom hinein und reichert sich in dem Atemgasstrom an. Das Anästhesiemittel-Reservoir bildet weiter eine Anästhesiemittelquelle für die Zuführeinrichtung. Die Zuführeinrichtung leitet das Anästhesiemittel aus dem Anästhesiemittel-Reservoir zur Verdunstungsoberfläche der Zuführeinrichtung, die der Messung der feuchten Temperatur durch den ersten Temperatursensor dient. Auf diese Weise ist ein ständiger Zufluss von Anästhesiemittel durch die Zuführeinrichtung zu der Verdunstungsoberfläche der Zuführeinrichtung gewährleistet.

Vorteilhafterweise ist die Zuführeinrichtung zumindest teilweise als poröser Festkörper, Sinterkörper, Vlies oder Faserstrumpf ausgebildet.

Auf diese Weise kann eine geringe Menge Anästhesiemittel in der Zuführeinrichtung gespeichert sein und von der Verdunstungsoberfläche verdunsten. Weiter kann die Zuführeinrichtung das Anästhesiemittel auf Grund der Kapillarwirkung von einer Anästhesiemittelquelle zu der Verdunstungsoberfläche transportieren. Alternativ kann die Zuführeinrichtung das Anästhesiemittel auch auf die Verdunstungsoberfläche aufsprühen.

Es ist weiter mit Vorteil eine Füllstandsüberwachung für die Verdunsterkammer vorgesehen, die ein Füllstandssignal an die Steuerungseinheit übermittelt. Mit einer Füllstandsüberwachung kann die Steuerungseinheit automatisch erkennen, wenn das Anästhesiemittel-Reservoir erschöpft ist. In diesem Fall muss das Anästhesiemittel-Reservoir aufgefüllt werden.

Es ist weiter vorteilhaft, wenn die Verdunsterkammer eine Einlassöffnung für das Atemgas aufweist, durch die das Atemgas als Atemgasstrom in die Verdunsterkammer einströmen kann.

Weiter ist es zweckmäßig, wenn eine Heizung stromaufwärts der Eingangsöffnung angeordnet ist, die den Atemgasstrom wärmt, bevor er in die Verdunsterkammer eintritt. Mit der Veränderung der Temperatur des eintretenden Atemgasstromes kann die Dosis des Anästhesiemittels, die in das Atemgas eingebracht wird, variiert werden. Die Heizung kann durch die Steuereinheit auf Basis der Anästhesiemittelkonzentration gesteuert werden.

Weiter kann zweckmäßigerweise eine Anästhesiemittel-Heizung von der Verdunsterkammer umfasst sein, die ausgebildet ist, das Anästhesiemittel in dem Anästhesiemittel-Reservoir zu erwärmen, wobei die Anästhesiemittel-Heizung vorzugsweise von der Steuerungseinheit gesteuert wird und vorzugsweise als Gasstromheizung oder Strahlungsheizung ausgebildet ist.

Mit der Änderung der Temperatur des Anästhesiemittels im Anästhesiemittel-Reservoir kann die Dosis des Anästhesiemittels, das in den Atemgasstrom eingebracht wird, ebenfalls variiert werden.

Weiter betrifft die Erfindung ein Verfahren zum Steuern und/oder Regeln einer Anästhesiemittelkonzentration in einem Atemgasstrom mittels einer Dosiervorrichtung für Anästhesiemittel gemäß einem der Ansprüche 1 bis 11, mit den Schritten: Erfassen einer Temperatur der Verdunstungsoberfläche mittels des ersten Temperatursensors; und Erfassen einer Temperatur des Atemgasstroms im Strömungskanal mittels des zweiten Temperatursensors, und Steuern und/oder Regeln einer Anästhesiemittelkonzentration im Atemgasstrom mittels der Temperatur des Atemgasstroms im Strömungskanal und der Temperatur der Verdunstungsoberfläche mittels der Steuerungseinheit der Dosiervorrichtung.

Zweckmäßigerweise weist das Verfahren die folgenden weiteren Schritte auf: Bestimmen der Anästhesiemittelkonzentration mittels der Temperatur der Verdunstungsoberfläche und der Temperatur des Atemgasstromes im Strömungskanals mittels eines Bestimmungsmoduls der Steuerungseinheit; und Regeln des Atemgasstroms auf Basis der Anästhesiemittelkonzentration mittels einer Regelungseinheit der Steuerungseinheit.

Vorteilhafterweise umfasst das Verfahren ein Bestimmen der Temperaturdifferenz zwischen der Temperatur der Verdunstungsoberfläche und der Temperatur des Atemgasstroms im Strömungskanal mittels eines Berechnungsmoduls der Steuerungseinheit; und ein Regeln der Anästhesiemittelkonzentration mittels der Dosiervorrichtung auf Basis der Temperaturdifferenz mittels einer Regelungseinheit.

Es kann auch als vorteilhaft angesehen werden, dass das Bestimmen der Anästhesiemittelkonzentration mittels der Temperaturen des Atemgasstroms und der Verdunstungsoberfläche und mittels eines Zustandsdiagramms für das Anästhesiemittel erfolgt.

Zu dem ersten und zweiten Temperatursignal wird mit Hilfe des Zustandsdiagramms ein zugehöriger Wert für die Konzentration bestimmt. Siehe hierzu analog die Ausführungen zur Dosiervorrichtung und beispielhaft die Ausführungen zu Figur 3. Die Konzentration kann damit ohne großen Aufwand schnell und genau bestimmt werden.

Hinsichtlich der Weiterbildungen des Verfahrens wird im Weiteren analog auf die oben angeführte Beschreibung zur Dosiervorrichtung verwiesen.

Im Folgenden wird anhand der beigefügten Zeichnungen ein Ausführungsbeispiel der Erfindung näher erläutert. Es zeigen:
- Fig. 1:: eine schematische Darstellung einer Dosiervorrichtung für Anästhesiemittel;
- Fig. 2a,b:: eine schematische Darstellung einer Verdunstungskammer mit Steuerungseinheit als Außendarstellung (a) und als schematische Schnittdarstellung (b); und
- Fig. 3:: eine beispielhafte Darstellung für ein Zustandsdiagramm eines Anästhesiemittels.

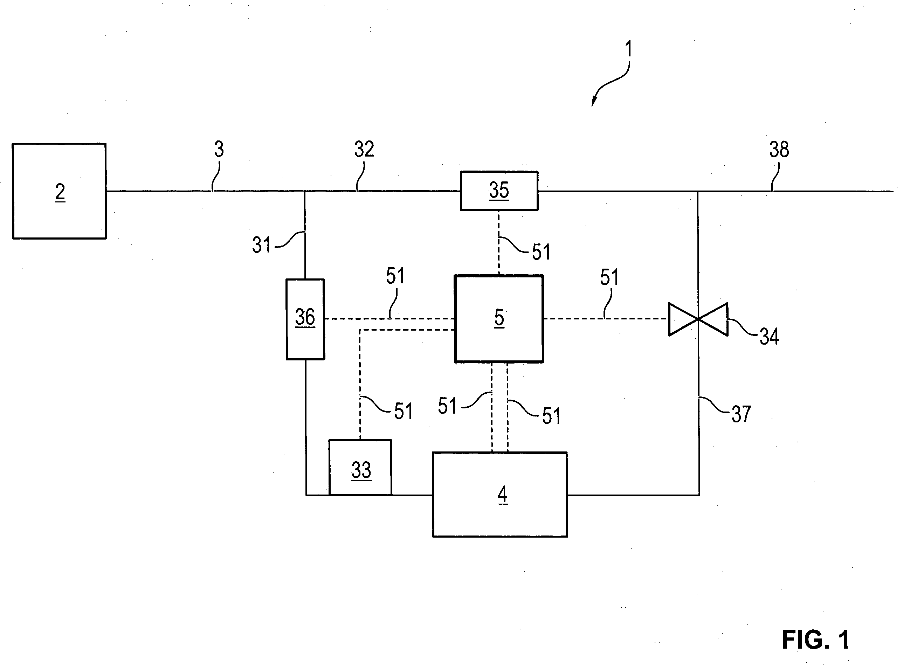

Die Dosiervorrichtung wird im Folgenden in ihrer Gesamtheit mit dem Referenzzeichen 1 bezeichnet. Figur 1 zeigt eine schematische Übersichtsdarstellung der Dosiervorrichtung 1. Die Dosiervorrichtung 1 stellt einem Patienten einen Atemgasstrom bereit, der ein Anästhesiemittel aufweist.

Zur Bereitstellung eines Atemgasstroms, der noch kein Anästhesiemittel umfasst, weist die Dosiervorrichtung 1 einen Gasmischer 2 auf. Der Atemgasstrom wird in dem Gasmischer 2 je nach Bedarf zusammengemischt und in ein Gasleitungssystem 3 eingeleitet.

Der Atemgasstrom wird über eine Zuführleitung 31 durch eine Einströmöffnung 41 in eine Verdunsterkammer 4 für ein Anästhesiemittel geführt. In der Verdunsterkammer 4 verdunstet Anästhesiemittel und wird von dem Atemgasstrom aufgenommen.

Von der Verdunsterkammer 4 leitet eine Verbindungsleitung 37 den mit Anästhesiemittel versetzten Atemgasstrom in eine Patientenzuleitung 38, die ihn weiter zum Patienten führt. Weiter ist eine Bypassleitung 32 vorgesehen, die den anästhesiemittelfreien Atemgasstrom von dem Gasmischer 2 an Zuführleitung 31, der Verdunsterkammer 4 und der Verbindungsleitung 37 vorbei zur Patientenzuleitung 38 führt. In der Patientenleitung 38 wird der Atemgasstrom aus der Bypassleitung 32, der kein Anästhesiemittel enthält, mit dem Atemgasstrom aus der Verbindungsleitung 37, der Anästhesiemittel enthält, vermischt. Die Konzentration des Anästhesiemittels in dem Atemgasstrom der Verbindungsleitung 37 sowie das Verhältnis zwischen den Atemgasströmen aus der Bypassleitung 32 und der Verbindungsleitung 37 determinieren die Konzentration des Anästhesiemittels in der Patientenleitung 38.

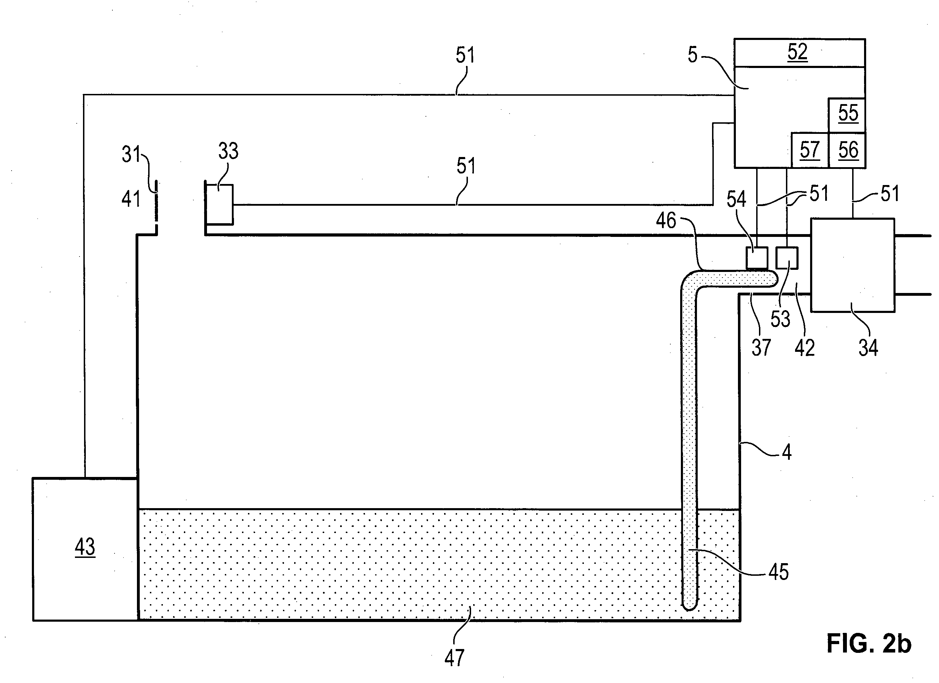

Gemäß Figur 2b weist die Verdunsterkammer 4 ein Anästhesiemittel-Reservoir 47 auf. In dem Anästhesiemittel-Reservoir 47 ist ein Anästhesiemittel angeordnet. Das Atemgas aus der Zuführleitung 31 wird über eine Einströmöffnung 41 in die Verdunsterkammer 4 eingeleitet und strömt über das offene Anästhesiemittel-Reservoir 47, d.h. die Oberfläche des darin gelagerten Anästhesiemittels. Der Atemgasstrom, der über das Anästhesiemittel-Reservoir 47 strömt, nimmt dabei das verdunstende Anästhesiemittel auf. Der mit Anästhesiemittel versetzte Atemgasstrom fließt weiter durch einen Strömungskanal 42 in die Verbindungsleitung 37. Der Strömungskanal 42 ist demnach stromabwärts des Anästhesiemittel-Reservoirs 47 angeordnet.

Weiter ist eine Zuführeinrichtung 45 vorgesehen, die eine Verdunstungsoberfläche 46 aufweist. Die Zuführeinrichtung 45 ist mit dem Anästhesiemittel-Reservoir 47 verbunden und fördert Anästhesiemittel von dem Anästhesiemittel-Reservoir 47 zu der Verdunstungsoberfläche 46.

Die Zuführeinrichtung 45 kann dabei als ein Docht ausgeführt sein. Das Dochtmaterial kann ein Vlies oder ein Faserstrumpf sein. Es können auch andere Materialien, die eine Kapillarwirkung haben, genutzt werden. Um die Verdunstung des Anästhesiemittels aus dem Dochtmaterial auf die Verdunstungsoberfläche 46 zu beschränken, kann die restliche Oberfläche der Zuführeinrichtung 45 mit einem Schutzmantel versehen sein, das z. B. als Metallrohr ausgestaltet sein kann.

Die Verdunstungsoberfläche 46 ist im Strömungskanal 42 angeordnet. Der Atemgasstrom, das mit Anästhesiemittel versetzt ist, streicht über die Verdunstungsoberfläche 46.

In einem ersten alternativen Betriebsmodus wird der Atemgasstrom beim Durchströmen der Verdunsterkammer 4 vollständig mit Anästhesiemittel gesättigt. Die Sättigung des Atemgasstroms mit Anästhesiemittel beträgt demnach 100 %. Der zu 100 % mit Anästhesiemittel gesättigte Atemgasstrom nimmt kein weiteres Anästhesiemittel von der Verdunstungsoberfläche 46 auf. Die Temperatur der Verdunstungsoberfläche 46 und die Temperatur des Atemgasstroms sind daher gleich, d.h. die Temperaturdifferenz zwischen der Verdunstungsoberfläche 46 und dem Abgasstrom ist minimal.

In einem zweiten alternativen Betriebsmodus wird der Atemgasstrom beim Durchströmen der Verdunsterkammer 4 nicht vollständig mit Anästhesiemittel gesättigt. Beim Überstreichen der Verdunstungsoberfläche 46 kühlt der nicht vollständig mit Anästhesiemittel gesättigte Atemgasstrom diese ab. Die Abkühlung erfolgt durch Verdunstung des Anästhesiemittels in den Atemgasstrom. Der nicht vollständig mit Anästhesiemittel gesättigte Atemgasstrom, der über die Verdunstungsoberfläche 46 streicht, nimmt damit also weiteres Anästhesiemittel über die Verdunstungsoberfläche 46 auf. Der Anteil des Atemgasstroms, der über die Verdunstungsoberfläche 46 streicht ist sehr klein. Im gesamten Atemgasstrom erfolgt daher keine signifikante Erhöhung der Anästhesiemittelkonzentration über die Verdunstungsoberfläche 46.

Die Abkühlung der Verdunstungsoberfläche 46 durch den nicht vollständig mit Anästhesiemittel gesättigten Atemgasstrom kann dabei nur so weit erfolgen, bis der Atemgasstrom, der über die Verdunstungsoberfläche 46 streicht, vollständig mit Anästhesiemittel gesättigt ist.

In beiden Betriebesmodi sind in dem Strömungskanal 42 ein erster Temperatursensor 54 und ein zweiter Temperatursensor 53 vorgesehen. Der zweite Temperatursensor 54 ermittelt die Temperatur des durch den Strömungskanal fließenden Atemgasstroms. Der erste Temperatursensor 53 ermittelt die Temperatur der Verdunstungsoberfläche 46. Thermische Schwankungen in der Verdunsterkammer 4 werden durch die Temperaturmessungen der Temperatursensoren 53, 54 automatisch berücksichtigt.

Der erste und der zweite Temperatursensor 53, 54 können in einer ersten alternativen Ausführungsform direkt in dem Strömungskanal angeordnet sein, wobei der erste Temperatursensor 54 in Kontakt mit der Verdunstungsoberfläche 46 stehen kann. In diesem Fall können die Temperatursensoren 53, 54 als elektrische Sensoren, gekapselte Thermoelemente, ungekapselte Thermoelemente, Widerstandselemente oder NTC-Widerstände ausgebildet sein.

In einer zweiten alternativen Ausführungsform können der erste und der zweite Temperatursensor 53, 54 die Temperatur auch kontaktlos ermitteln. In dieser Ausführungsform kann mindestens einer der Temperatursensoren 53, 54 als ein Infrarotdetektor ausgestaltet sein.

In einer dritten alternativen Ausführungsform können der erste und der zweite Temperatursensor 53, 54 auf einem einstückigen kontaktlos messenden Temperatursensor zusammengefasst sein. Dabei kann der einstückige Temperatursensor als ortsauflösender Infrarotsensor ausgestaltet sein, der die Verdunstungsoberfläche 46 und den Atemgasstrom in einem Abstand von der Verdunstungsoberfläche 46 beobachtet. Der Teil des ortsauflösenden Infrarotsensors, der die Verdunstungsoberfläche beobachtet, ist dabei als der erste Temperatursensor 54 definiert, wobei der andere Teil des ortsauflösenden Infrarotsensors, der den trockenen Atemgasstrom beobachtet, als der zweite Temperatursensor 53 definiert ist.

Der erste und der zweite Temperatursensor 53, 54 erzeugen Temperatursignale T1, T2, die über Signalleitungen 51 an eine Steuerungseinheit 5 übermittelt werden.

Die Steuerungseinheit 5 weist für den ersten Betriebsmodus ein Berechnungsmodul 56 auf, das die Temperaturdifferenz zwischen dem ersten und dem zweiten Temperatursignal T1, T2 bestimmt. Weiter weist die Steuerungseinheit 5 eine Regelungseinheit 57 auf, an die das Berechnungsmodul 56 ein Temperaturdifferenzsignal übermittelt. In dem ersten Betriebsmodus ist die Regelungseinheit 57 dazu ausgebildet, die Temperaturdifferenz zu minimieren, im Idealfall auf Null zu regeln. Die Steuerungseinheit 5 gibt der Regelungseinheit 57 dabei eine Führungsgröße vor.

Die Steuerungseinheit 5 ermittelt aus den Temperatursignalen der Temperatursensoren 53, 54 weiter die Konzentration des Anästhesiemittels in dem Atemgasstrom. Dazu hat die Steuerungseinheit 5 Zugriff auf ein h+,x-Diagramm nach Mollier des betreffenden Anästhesiemittels. Das h+,x-Diagramm nach Mollier kann in einem Bestimmungsmodul 52 gespeichert sein, das mit der Steuerungseinheit 5 so verbunden ist, so dass die Steuerungseinheit 5 das Bestimmungsmodul 52 auslesen kann. Alternativ kann auch ein Carrier-Diagramm verwendet werden. Weiter kann alternativ auch ein formelmäßiger Zusammenhang oder ein anderes geeignetes Zustandsdiagramm des benutzten Anästhesiemittels zur Bestimmung der Anästhesiemittelkonzentration in dem Atemgas verwendet werden.

In dem zweiten Betriebsmodus ist die Regelungseinheit 57 dazu ausgebildet, die Konzentration des Anästhesiemittels im Atemgasstrom auf Basis der Temperaturdaten zu regeln. Die Temperaturdaten können dabei aus dem Temperatursignal T1 des ersten Temperatursensors 54 oder aus der Differenz der Temperatursignale T1, T2 des ersten und des zweiten Temperatursensors 53, 54 ermittelt werden.

Die Regelungseinheit 57 ist über eine zusätzliche Signalleitung 51 mit einem Ausström-Ventil 34 verbunden, das in der Verbindungsleitung 37 angeordnet ist. Das Ausström-Ventil 34 kann die Verbindungsleitung 37 sperren oder öffnen. Weiter kann das Ausström-Ventil 34 eine teilweise Öffnung der Verbindungsleitung 37 bewirken.

Das Ausström-Ventil 34 kann dabei als Proportional-Ventil ausgebildet sein. Dabei können auch verschiedene Untervarianten des Proportional-Ventils, zum Beispiel ein Konus-Ventil, verwendet werden.

Das Ausström-Ventil 34 übermittelt seinen Öffnungszustand über eine Signalleitung 51 an die Steuerungseinheit 5. Die Steuerungseinheit 5 ermittelt aus der ermittelten Anästhesiemittelkonzentration im Atemgasstrom einen Öffnungs-Sollwert für das Ausström-Ventil 34. Der Öffnungs-Sollwert wird an das Ausström-Ventil 34 übermittelt, wenn er von dem Öffnungs-Istwert des Ausström-Ventils 34 abweicht. Auf diese Weise kann das Mischungsverhältnis zwischen den Atemgasströmen aus der Bypassleitung 32 und der Verbindungsleitung 37 gesteuert werden. Auf diese Weise kann damit die Anästhesiemittelkonzentration im Atemgasstrom in der Patientenzuleitung 38 gesteuert werden.

Über einen Sollwert-Eingang 55 erhält die Steuerungseinheit 5 von dem Bedienpersonal der Dosiervorrichtung 1 ein Sollwert-Signal für die Anästhesiemittelkonzentration im Atemgas. Auf Basis des eingegebenen Sollwerts und der ermittelten Anästhesiemittelkonzentration im Atemgasstrom wird die Öffnung des Ausström-Ventils 34 von der Steuerungseinheit 5 gesteuert.

Die Steuerungseinheit 5 kann weiter über Signalleitungen 51 mit den Strömungswiderständen 35, 36 in dem Gasleitungssystem 3 verbunden sein. So kann ein erster Strömungswiderstand 35 in der Bypassleitung 32 vorgesehen sein, wobei der erste Strömungswiderstand 35 durch die Steuerungseinheit 5 gesteuert werden kann. Damit kann der Atemgasstrom in der Bypassleitung 32 von der Steuerungseinheit 5 mittels des Strömungswiderstands 35 so gesteuert werden, dass eine Soll-Anästhesiemittelkonzentration nach der Durchmischung mit dem Atemgasstrom aus der Verbindungsleitung 37 von der Verdunsterkammer 4 erfolgt.

Es kann weiter ein zweiter steuerbarer Strömungswiderstand 36 in der Zuführleitung 31 zur Verdunsterkammer 4 vorgesehen sein. Der zweite Strömungswiderstand 36 ist ebenfalls über eine Signalleitung 51 mit der Steuerungseinheit 5 verbunden. Auf diese Weise kann die Steuerungseinheit 5 den Atemgasstrom im gesamten Gasleitungssystem 3 beeinflussen.

Weiter kann eine Leitungsheizung 33 an der Zuführleitung 31 zur Verdunsterkammer 4 vorgesehen sein, die den durch die Zuführleitung 31 fließenden Atemgasstrom erwärmt. Die Leitungsheizung 33 kann über eine Signalleitung 51 mit der Steuerungseinheit 5 verbunden sein, sodass die Regelungseinheit 57 die Leitungsheizung 33 regeln kann. Die Steuerungseinheit 5 kann über die Regelungseinheit 57 damit die Temperatur des durch die Verdunsterkammer 4 strömenden Atemgasstroms beeinflussen und damit auch die Dosis des Anästhesiemittels, die in den Atemgasstrom aufgenommen wird, beeinflussen.

Weiter kann eine Anästhesiemittel-Heizung 43 vorgesehen sein, die am Anästhesiemittel-Reservoir 47 angeordnet ist. Die Anästhesiemittel-Heizung 43 ist dazu ausgebildet das Anästhesiemittel im Anästhesiemittel-Reservoir 47 zu erwärmen. Auch die Anästhesiemittel-Heizung 43 kann über eine Signalleitung 51 mit der Regelungseinheit 57 verbunden sein, sodass die Steuerungseinheit 5 über die Regelungseinheit 57 die Temperatur des Anästhesiemittels im Anästhesiemittel-Reservoir 47 beeinflussen kann. Auch mit der Anästhesiemittel-Heizung 43 kann die Dosis des Anästhesiemittels, das in den Atemgasstrom verdunstet, durch die Steuerungseinheit 5 beeinflusst werden.

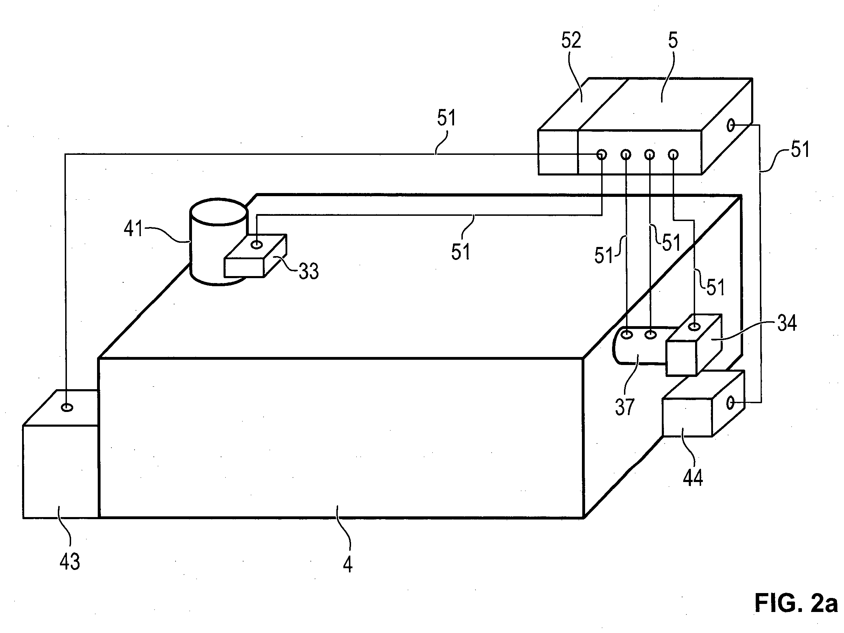

Gemäß Figur 2a ist weiter eine Füllstandsüberwachung 44 vorgesehen, die den Füllstand des Anästhesiemittels im Anästhesiemittel-Reservoir 47 überwacht. Die Füllstandsüberwachung 44 überwacht den Füllstand des Anästhesiemittel-Reservoirs 47 automatisch und übermittelt über eine Signalleitung 51 ein Füllstandssignal an die Steuerungseinheit 5. Die Steuerungseinheit 5 kann auf diese Weise ermitteln, ob das Anästhesiemittel-Reservoir 47 leer ist oder nicht. Wenn das Anästhesiemittel-Reservoir 47 geleert ist, kann die Steuerungseinheit 5 ausgebildet sein, ein Signal an die Benutzer der Dosiervorrichtung 1 auszugeben, das einen Nachfüllbedarf des Anästhesiemittel-Reservoirs 47 meldet. Weiter kann die Steuerungseinheit 5 in diesem Fall auch die Zuführleitung 31 und die Verbindungsleitung 37 des Gasleitungssystems 3 sperren und damit den Strom des Atemgases durch die Verdunsterkammer 4 unterbinden. Im Weiteren wird dann die Erfassung der Temperaturen mittels der Temperatursensoren 53, 54 gestoppt, da eine Verdunstung von Anästhesiemittel an der Verdunstungsoberfläche 46 nicht mehr stattfinden kann. Weiter ist dann auch kein Anästhesiemittel mehr im Atemgasstrom vorhanden, sodass eine Konzentrationsbestimmung sich erübrigt.

Im Folgenden wird die Bestimmung der Konzentration des Anästhesiemittels im Atemgasstrom mittels eines h+,x-Diagramm nach Mollier beispielhaft beschrieben.

Ein h+,x-Diagramm 6 nach Mollier ist in Figur 3 abgebildet. Aufgetragen ist die Temperatur über die Konzentration. Eingetragen ist die 100%-Sättigungskurve 7, die eine 100-prozentige Sättigung des Atemgasstroms mit Anästhesiemittel angibt. Unterhalb dieser Kurve ist der Nebelbereich, in dem sich das Anästhesiemittel als Nebel im Atemgasstrom manifestieren würde. Oberhalb der 100%-Sättigungskurve 7 bildet sich kein Nebel. Je weiter weg sich ein Zustandspunkt von der 100%-Sättigungskurve 7 im oberen Bereich befindet, desto geringer ist die Sättigung des Atemgasstroms mit Anästhesiemittel.

Zur Veranschaulichung ist weiter die 50%-Sättigungskurve 8 eingetragen, die eine 50-prozentige Sättigung des Atemgasstroms mit Anästhesiemittel abbildet.

Weiter eingetragen sind die sogenannten Nebelisothermen 9, die näherungsweise adiabate Zustandsänderungen in dem Diagramm beschreiben. Entlang einer Nebelisothermen 9 ist die Enthalpie näherungsweise konstant. Die Nebelisothermen 9 sind in der Regel sehr steil, d. h. sie verlaufen nahezu senkrecht. Eine Änderung der Temperatur resultiert daher in einer nahezu gleichen Konzentration des Anästhesiemittels im Atemgasstrom.

Mittels des ersten Temperatursensors 54 wird die Temperatur der Verdunstungsoberfläche 46 im Strömungskanal 42 ermittelt, die eine "feuchte" Temperatur ist. Dies resultiert daher, dass die Zuführeinrichtung 45 mit dem Anästhesiemittel befeuchtet ist, wobei das Anästhesiemittel von der Verdunstungsoberfläche 46 in den Atemgasstrom verdunstet. Diese erste Temperatur ist im Diagramm 6 mit T1 eingetragen.

Da die erste Temperatur T1 auf der durch den Atemgasstrom gekühlten Verdunstungsoberfläche 46 gemessen wird, entspricht die Temperatur der Verdunstungsoberfläche 46 der Temperatur des Atemgasstromes, der in direktem Kontakt mit der Verdunstungsoberfläche 46 steht. Der Atemgasstrom, der in direktem Kontakt mit der Verdunstungsoberfläche 46 steht, ist zu 100 % mit Anästhesiemittel gesättigt. Daher ist der Zustandspunkt des mit Anästhesiemittel gesättigten Atemgases im Diagramm 6 bekannt. Dieser Zustandspunkt ist im Diagramm 6 mit dem Referenzzeichen 12 bezeichnet und liegt auf der Kurve 7.

Das Anästhesiemittel und der Atemgasstrom ändern den Zustand adiabatisch, wenn sie sich von der Verdunstungsoberfläche 46 wegbewegen, d. h. bei der Zustandsänderung findet kein Wärmeaustausch mit der Umgebung statt. Die Zustandsänderung erfolgt daher näherungsweise entlang der Nebelisothermen 9'. Dies ist durch den Pfeil 10 gekennzeichnet.

Die Steuerungseinheit 5 ermittelt mit dem zweiten Temperatursensor 53 eine zweite Temperatur im Strömungskanal 42, die der "trockenen" Temperatur des Atemgasstroms entspricht, wobei der Atemgasstrom eine bestimmte Anästhesiemittelkonzentration aufweist. Diese Temperatur ist im Diagramm 6 als T2 eingetragen.

Zwischen dem Atemgasstrom an der Verdunstungsoberfläche 46 und dem trockenen Atemgasstrom kann eine adiabate Zustandsänderung angenommen werden. Daher müssen beide Zustandspunkte auf der gleichen Adiabaten liegen, die durch die Nebelisotherme angenähert ist. Der trockene Atemgasstrom hat daher einen Zustand, der auf dieser Nebelisothermen 9' angeordnet ist. Mit der ermittelten Temperatur T2 des Atemgasstroms und der Nebelisothermen 9' kann auf diese Weise der Zustandspunkt des mit Anästhesiemittel beaufschlagten Atemgasstroms berechnet werden. Die Zustandsänderung ist mit dem Pfeil 10 hervorgehoben. In Kenntnis der Temperatur T2 kann damit die genaue Konzentration des Anästhesiemittels im Atemgasstrom mittels des durch die Steuerungseinheit 5 bestimmten Zustandspunktes ermittelt werden (siehe Pfeil 11).

Da der Steuerungseinheit 5 nun die Anästhesiemittelkonzentration im Atemgasstrom bekannt ist, kann die Steuerungseinheit 5 ermitteln, wie das Mischungsverhältnis zwischen dem Atemgasstrom in der Bypassleitung 32 und dem Atemgasstrom mit Anästhesiemittel in der Verbindungsleitung 37 von der Verdunsterkammer 4 beschaffen sein muss, um den Sollwert für die Konzentration des Anästhesiemittels zu erreichen. Aufgrund dieser Werte steuert die Steuerungseinheit 5 das Ausström-Ventil 34 an. Weiter können die Strömungswiderstände 35, 36 sowie die Heizungen 33 und 43 so angesteuert werden, dass sich die entsprechende Anästhesiemittelkonzentration im Atemgasstrom in der Patientenzuleitung 38 einstellt.

Die Berechnung der Anästhesiemittelkonzentration im Atemgasstrom der Patientenzuleitung 38 kann durch die Steuerungseinheit 5 mittels höchstens drei stoffspezifischen Kalibrierkonstanten in Echtzeit erfolgen.

### Bezugszeichenliste

- 1: Dosiervorrichtung
- 2: Gasmischer
- 3: Gasleitungssystem
- 31: Zuführleitung
- 32: Bypassleitung
- 33: Leitungsheizung
- 34: Ausström-Ventil
- 35: erster Strömungswiderstand
- 36: zweiter Strömungswiderstand
- 37: Verbindungsleitung
- 38: Patientenzuleitung

- 4: Verdunsterkammer
- 41: Einströmöffnung
- 42: Strömungskanal
- 43: Anästhesiemittel-Heizung
- 44: Füllstandsüberwachung
- 45: Zuführeinrichtung
- 46: Verdunstungsoberfläche
- 47: Anästhesiemittel-Reservoir

- 5: Steuerungseinheit
- 51: Signalleitung
- 52: Bestimmungsmodul
- 53: zweiter Temperatursensor
- 54: erster Temperatursensor
- 55: Sollwert-Eingang
- 56: Berechnungsmodul
- 57: Regelungseinheit
- 6: h+,x-Diagramm
- 7: Sättigungskurve 100 %
- 8: Sättigungskurve 50 %
- 9: Nebelisotherme
- 10: erster Pfeil
- 11: zweiter Pfeil
- 12: Zustandspunkt

## Patentansprüche

1. Dosiervorrichtung (1) für Anästhesiemittel in einem Atemgasstrom, die einen Strömungskanal (42) für den das Anästhesiemittel aufweisenden Atemgasstrom, eine Steuerungseinheit (5) und einen ersten Temperatursensor (54)aufweist, wobei die Dosiervorrichtung (1) eine Zuführeinrichtung (45) mit einer im Strömungskanal (42) angeordneten Verdunstungsoberfläche (46) für Anästhesiemittel aufweist, wobei der erste Temperatursensor (54) die Temperatur der Verdunstungsoberfläche (46) erfasst und ein erstes Temperatursignal (T1) an die Steuerungseinheit (5) übermittelt, **dadurch gekennzeichnet, dass** die Dosiervorrichtung (1) einen zweiten Temperatursensor (53) aufweist, der die Temperatur des Atemgasstroms im Strömungskanal (42) erfasst und als zweites Temperatursignal (T2) an die Steuerungseinheit (5) übermittelt, und wobei die Steuerungseinheit (5) zum Steuern einer Anästhesiemittelkonzentration mittels des ersten und zweiten Temperatursignals (T1, T2) ausgebildet ist.

2. Dosiervorrichtung nach Anspruch 1, **dadurch gekennzeichnet, dass** die Steuerungseinheit (5) ein Bestimmungsmodul (52) aufweist, das aus dem ersten und zweiten Temperatursignal einen Zustandspunkt des Anästhesiemittels bestimmt, wobei der Zustandspunkt einer bestimmten Anästhesiemittelkonzentration im Atemgasstrom zugeordnet ist.

3. Dosiervorrichtung nach einem der Ansprüche 1 oder 2, **dadurch gekennzeichnet, dass** die Steuerungseinheit (5) ein Berechnungsmodul (56) aufweist, das zum Bestimmen einer Differenz zwischen den Temperatursignalen (T1, T2) ausgebildet ist, wobei die Steuerungseinheit (5) zum Steuern der Anästhesiemittelkonzentration mittels des ersten und zweiten Temperatursignals (T1, T2) ausgebildet ist.

4. Dosiervorrichtung nach Anspruch 3, **dadurch gekennzeichnet, dass** die Steuerungseinheit (5) eine Regelungseinheit (57) aufweist, die zum Regeln des Differenzbetrags auf ein Minimum ausgebildet ist.

5. Dosiervorrichtung nach einem der Ansprüche 1 bis 4, **dadurch gekennzeichnet, dass** die Steuerungseinheit (5) einen Sollwerteingang (55) für ein Sollwertsignal für die Anästhesiemittelkonzentration im Atemgasstrom aufweist und eine bzw. die Regelungseinheit (57) aufweist, die zum Regeln der Anästhesiemittelkonzentration in einer Patientenzuleitung (38) auf das Sollwertsignal ausgebildet ist.

6. Dosiervorrichtung nach einem der vorangegangenen Ansprüche, **dadurch gekennzeichnet, dass** die Dosiervorrichtung (1) ein Ausström-Ventil (34) aufweist, das fluidkommunizierend mit dem Strömungskanal (42) verbunden ist und durch die Regelungseinheit (57) geregelt wird.

7. Dosiervorrichtung nach Anspruch 6 , **dadurch gekennzeichnet, dass** das Ausström-Ventil (34) als ein Proportional-Ventil, vorzugsweise als ein Konus-Ventil, ausgebildet ist.

8. Dosiervorrichtung nach einem der vorangegangenen Ansprüche, **dadurch gekennzeichnet, dass** der erste Temperatursensoren (54) als ein elektrischer Sensor, ein gekapseltes Thermoelement, ein ungekapseltes Thermoelement, ein Widerstandselement, und/oder als NTC-Widerstand ausgebildet ist.

9. Dosiervorrichtung nach einem der vorangegangenen Ansprüche, **dadurch gekennzeichnet, dass** der erste Temperatursensoren (54) als kontaktlos messender Infrarotdetektor ausgebildet ist.

10. Dosiervorrichtung nach einem der vorangegangenen Ansprüche, **dadurch gekennzeichnet, dass** die Temperatursensoren (53, 54) einstückig als kontaktlos messender ortsauflösender Infrarotsensor ausgebildet sind.

11. Dosiervorrichtung nach einem der vorangegangenen Ansprüche, **dadurch gekennzeichnet, dass** die Dosiervorrichtung (1) eine Verdunsterkammer (4) aufweist, in der stromaufwärts der Verdunstungsoberfläche (46) ein Anästhesiemittel-Reservoir (47) angeordnet ist, wobei die Zuführeinrichtung (45) mit dem Anästhesiemittel-Reservoir (47) fluidkommunizierend verbunden ist.

12. Verfahren zum Steuern und/oder Regeln einer Anästhesiemittelkonzentration in einem Atemgasstroms mittels einer Dosiervorrichtung (1) für Anästhesiemittel nach einem der Ansprüche 1 bis 11, mit den Schritten: Erfassen einer Temperatur der Verdunstungsoberfläche (46) mittels des ersten Temperatursensors (54); und Erfassen einer Temperatur des Atemgasstroms im Strömungskanal (42) mittels des zweiten Temperatursensors (53), und Steuern und/oder Regeln einer Anästhesiemittelkonzentration im Atemgasstrom mittels der Temperatur der Verdunstungsoberfläche (46) und der Temperatur des Atemgasstromes im Strömungskanal mittels der Steuerungseinheit (5) der Dosiervorrichtung.

13. Verfahren nach Anspruch 12, **gekennzeichnet durch** Bestimmen der Anästhesiemittelkonzentration mittels der Temperatur der Verdunstungsoberfläche (46) und der Temperatur des Atemgasstromes im Strömungskanals mittels eines Bestimmungsmoduls (52) der Steuerungseinheit (5); und Regeln des Atemgasstroms auf Basis der Anästhesiemittelkonzentration mittels einer Regelungseinheit (57) der Steuerungseinheit (5).

14. Verfahren nach einem der Ansprüche 12 oder 13, **gekennzeichnet durch** Bestimmen der Temperaturdifferenz zwischen der Temperatur der Verdunstungsoberfläche (46) und der Temperatur des Atemgasstroms im Strömungskanal (42) mittels eines Berechnungsmoduls (56) der Steuerungseinheit (5); und Regeln der Anästhesiemittelkonzentration mittels der Dosiervorrichtung (1) auf Basis der Temperaturdifferenz mittels einer Regelungseinheit (57).

15. Verfahren nach einem der Ansprüche 12 bis 14, **dadurch gekennzeichnet, dass** das Bestimmen der Anästhesiemittelkonzentration mittels der Temperaturen des Atemgasstroms und der Verdunstungsoberfläche (46) und mittels eines Zustandsdiagramms für das Anästhesiemittel erfolgt.

## Claims

1. Metering device (1) for anaesthetic in a respiratory gas flow, having a flow channel (42) for the respiratory gas flow containing the anaesthetic, a control unit (5) and a first temperature sensor (54), wherein the metering device (1) has a feed device (45) with an anaesthetic evaporation surface (46) arranged in the flow channel (42), wherein the first temperature sensor (54) detects the temperature of the evaporation surface (46) and transmits a first temperature signal (T1) to the control unit (5), **characterized in that** the metering device (1) has a second temperature sensor (53), which detects the temperature of the respiratory gas flow in the flow channel (42) and transmits a second temperature signal (T2) to the control unit (5), and wherein the control unit (5) is configured to control an anaesthetic concentration by means of the first temperature signal (T1) and second temperature signal (T2).

2. Metering device according to Claim 1, **characterized in that** the control unit (5) has a determination module (52) which, from the first and second temperature signal, determines a phase point of the anaesthetic, wherein the phase point is assigned to a defined anaesthetic concentration in the respiratory gas flow.

3. Metering device according to either of Claims 1 and 2, **characterized in that** the control unit (5) has a calculation module (56), which is configured to determine a difference between the temperature signals (T1, T2), wherein the control unit (5) is configured to control the anaesthetic concentration by means of the first temperature signal (T1) and second temperature signal (T2).

4. Metering device according to Claim 3, **characterized in that** the control unit (5) has a regulating unit (57), which is configured to regulate the difference amount to a minimum.

5. Metering device according to one of Claims 1 to 4, **characterized in that** the control unit (5) has a setpoint value input (55) for a setpoint value signal for the anaesthetic concentration in the respiratory gas flow and has a or the regulating unit (57), which is configured to regulate the anaesthetic concentration in a patient feed line (38) to the setpoint value signal.

6. Metering device according to one of the preceding claims, **characterized in that** the metering device (1) has an outflow valve (34), which is connected to the flow channel (42) in a fluid-communicating manner and is regulated by the regulating unit (57).

7. Metering device according to Claim 6, **characterized in that** the outflow valve (34) is configured as a proportional valve, preferably as a cone valve.

8. Metering device according to one of the preceding claims, **characterized in that** the first temperature sensor (54) is configured as an electric sensor, an encapsulated thermocouple, a non-encapsulated thermocouple, a resistor element, and/or as an NTC resistor.

9. Metering device according to one of the preceding claims, **characterized in that** the first temperature sensor (54) is configured as an infrared detector for contactless measurement.

10. Metering device according to one of the preceding claims, **characterized in that** the temperature sensors (53, 54) are configured in one piece as a spatially resolving infrared sensor for contactless measurement.

11. Metering device according to one of the preceding claims, **characterized in that** the metering device (1) has an evaporator chamber (4), in which an anaesthetic reservoir (47) is arranged upstream from the evaporation surface (46), wherein the feed device (45) is connected to the anaesthetic reservoir (47) in a fluid-communicating manner.

12. Method for controlling and/or regulating an anaesthetic concentration in a respiratory gas flow by means of a metering device (1) for anaesthetic according to one of Claims 1 to 11, with the steps of: detecting a temperature of the evaporation surface (46) by means of the first temperature sensor (54), detecting a temperature of the respiratory gas flow in the flow channel (42) by means of the second temperature sensor (53), and controlling and/or regulating an anaesthetic concentration in the respiratory gas flow by means of the temperature of the evaporation surface (46) and the temperature of the respiratory gas flow in the flow channel by means of the control unit (5) of the metering device.

13. Method according to Claim 12, **characterized by** determining the anaesthetic concentration by means of the temperature of the evaporation surface (46) and the temperature of the respiratory gas flow in the flow channel by means of a determination module (52) of the control unit (5), and regulating the respiratory gas flow on the basis of the anaesthetic concentration by means of a regulating unit (57) of the control unit (5).

14. Method according to either of Claims 12 and 13, **characterized by** determining the temperature difference between the temperature of the evaporation surface (46) and the temperature of the respiratory gas flow in the flow channel (42) by means of a calculation module (56) of the control unit (5), and regulating the anaesthetic concentration by means of the metering device (1) on the basis of the temperature difference by means of a regulating unit (57).

15. Method according to one of Claims 12 to 14, **characterized in that** the determination of the anaesthetic concentration is effected by means of the temperatures of the respiratory gas flow and of the evaporation surface (46) and by means of a phase diagram for the anaesthetic.

## Revendications

1. Dispositif de dosage (1) d'agent anesthésique dans un flux de gaz respiratoire, comportant un canal d'écoulement (42) pour le flux de gaz respiratoire comprenant l'agent anesthésique, une unité de commande (5) et un premier capteur de température (54), dans lequel le dispositif de dosage (1) comporte un dispositif d'alimentation (45) doté d'une surface d'évaporation (46) disposée dans le canal d'écoulement (42), pour ledit agent anesthésique, le premier capteur de température (54) détectant la température de la surface d'évaporation (46) et transmettant un premier signal de température (T1) à l'unité de commande (5), **caractérisé en ce que** le dispositif de dosage (1) comprend un deuxième capteur de température (53) qui détecte la température du flux de gaz respiratoire dans le canal d'écoulement (42) et la transmet en tant que deuxième signal de température (T2) à l'unité de commande (5), et l'unité de commande (5) étant réalisée pour commander une concentration de l'agent anesthésique au moyen des premier et deuxième signaux de température (T1, T2).

2. Dispositif de dosage selon la revendication 1, **caractérisé en ce que** l'unité de commande (5) comporte un module de détermination (52) qui détermine un point d'état de l'agent anesthésique à partir des premier et deuxième signaux de températures, le point d'état étant associé à une concentration déterminée de l'agent anesthésique dans le flux de gaz respiratoire.

3. Dispositif de dosage selon l'une des revendications 1 ou 2, **caractérisé en ce que** l'unité de commande (5) comporte un module de calcul (56) qui est réalisé pour déterminer une différence entre les signaux de température (T1, T2), l'unité de commande (5) étant réalisée pour commander la concentration de l'agent anesthésique au moyen des premier et deuxième signaux de température (T1, T2).

4. Dispositif de dosage selon la revendication 3, **caractérisé en ce que** l'unité de commande (5) comprend une unité de régulation (57) qui est réalisée pour réguler au minimum la valeur de la différence.

5. Dispositif de dosage selon l'une des revendications 1 à 4, **caractérisé en ce que** l'unité de commande (5) comporte une entrée de valeur de consigne (55) destinée à un signal de valeur de consigne pour la concentration de l'agent anesthésique dans le flux de gaz respiratoire et comporte une ou ladite unité de commande (57), qui est réalisée pour réguler conformément au signal de valeur de consigne la concentration de l'agent anesthésique dans un conduit d'alimentation de patient (38).

6. Dispositif de dosage selon l'une des revendications précédentes, **caractérisé en ce que** le dispositif de dosage (1) comporte une soupape d'écoulement de sortie (34) qui est reliée au canal d'écoulement (42) et est régulée par communication fluidique par l'unité de régulation (57).

7. Dispositif de dosage selon la revendication 6, **caractérisé en ce que** la soupape d'écoulement de sortie (34) est réalisée sous la forme d'une soupape proportionnelle, de préférence sous la forme d'une soupape conique.

8. Dispositif de dosage selon l'une des revendications précédentes, **caractérisé en ce que** le premier capteur de température (54) est réalisé sous la forme d'un capteur électrique, d'un thermocouple encapsulé, d'un thermocouple non encapsulé, d'un élément résistif et/ou d'une résistance NTC.

9. Dispositif de dosage selon l'une des revendications précédentes, **caractérisé en ce que** le premier capteur de température (54) est réalisé sous la forme d'un détecteur infrarouge à mesure sans contact.

10. Dispositif de dosage selon l'une des revendications précédentes, **caractérisé en ce que** les capteurs de température (53, 54) sont réalisés en une seule pièce sous la forme d'un capteur infrarouge à mesure sans contact et à résolution spatiale.

11. Dispositif de dosage selon l'une des revendications précédentes, **caractérisé en ce que** le dispositif de dosage (1) comporte une chambre d'évaporation (4) dans laquelle un réservoir d'agent anesthésique (47) est disposé en amont de la surface d'évaporation (46), le dispositif d'alimentation (45) étant relié par communication fluidique au réservoir d'agent anesthésie (47).

12. Procédé de commande et/ou de régulation d'une concentration d'agent anesthésique dans un flux de gaz respiratoire au moyen d'un dispositif de dosage (1) destiné à un agent anesthésique selon l'une des revendications 1 à 11, comprenant les étapes suivantes : la détection d'une température de la surface d'évaporation (46) par ledit premier capteur de température (54) ; et la détection d'une température du flux de gaz respiratoire dans le canal d'écoulement (42) au moyen du deuxième capteur de température (53), et la commande et/ou la régulation d'une concentration de l'agent anesthésique dans l'écoulement de gaz respiratoire au moyen de la température de la surface d'évaporation (46), et de la température du flux de gaz respiratoire dans le canal d'écoulement au moyen de l'unité de commande (5) du dispositif de dosage.

13. Procédé selon la revendication 12, **caractérisé par** la détermination de la concentration de l'agent anesthésique au moyen de la température de la surface d'évaporation (46), et de la température du flux de gaz respiratoire dans le canal d'écoulement au moyen d'un module de détermination (52) de l'unité de commande (5) ; et par la régulation du flux de gaz respiratoire sur la base de la concentration de l'agent anesthésique au moyen d'une unité de régulation (57) de l'unité de commande (5).

14. Procédé selon l'une des revendications 12 ou 13, **caractérisé par** la détermination de la différence de température entre la température de la surface d'évaporation (46) et la température du flux de gaz respiratoire dans le canal d'écoulement (42) au moyen d'un module de calcul (56) de l'unité de commande (5) ; et par la régulation de la concentration de l'agent anesthésique au moyen du dispositif de dosage (1) sur la base de la différence de température au moyen d'une unité de régulation (57).

15. Procédé selon l'une des revendications 12 à 14, **caractérisé en ce que** la concentration de l'agent anesthésique est déterminée au moyen des températures du flux de gaz respiratoire et de la surface d'évaporation (46) et au moyen d'un diagramme d'état de l'agent anesthésique.
